# FASCICULE DE BREVET EUROPEEN

(11) **EP 0 595 988 B1**
(45) Date de publication et mention de la délivrance du brevet: **27.12.1995**
(21) Numéro de dépôt: 92916472.1
(22) Date de dépôt: 17.07.1992
(51) Int. Cl.: A61K 7/48, A61K 31/365, A61K 35/78

(54) **COMPOSITIONS COSMETIQUES CONTENANT UN EXTRAIT D'ECLIPTA ALBA ------------------------------------------------------------**
KOSMETISCHE ZUSAMMENSETZUNGEN, DIE EINEN EXTRAKT AUS ECLIPTA ALBA ENTHALTEN
COSMETIC COMPOSITIONS COMPRISING AN EXTRACT OF ECLIPTA ALBA

(30) Priorité: 19.07.1991 FR 9109184
(43) Date de publication de la demande: 11.05.1994
(73) Titulaire: SABLON, Louis Emmanuel, F-97180 Sainte-Anne (FR)
(72) Inventeur: SABLON, Louis Emmanuel, F-97180 Sainte-Anne (FR)
(74) Mandataire: Ores, Irène
(86) Numéro de dépôt international: FR9200699
(87) Numéro de publication internationale: WO9301796

(56) Documents cités:
- DE-A- 3 525 363
- STN Serveur de Bases de Donnees, Karlsruhe DE, Fichier Chemical Abstracts, vol. 113, no. 12, (Columbus, Ohio, US), voir résumé no. 103209f, & CN,A,1031022 (TIANJIN INSTITUTE OF LIGHT INDUSTRIAL CHEMISTRY) 15 Février 1989, voir résumé

## Description

L'Invention est relative à de nouvelles compositions cosmétiques permettant la régulation de la pigmentation cutanée.

Les irrégularités de la pigmentation de la peau ont toujours été considérées comme inesthétiques, et depuis très longtemps, différents moyens d'y remédier ont été proposés.

Ces défauts se manifestent le plus souvent sous forme de taches d'hyperpigmentation, qui peuvent prendre des formes diverses : taches de rousseur, chloasma d'origine hormonale, lentigo sénile siégeant principalement sur les mains, hyperpigmentation cicatricielle ou provoquée par un agent photosensibilisant, etc...

Les solutions les plus diverses ont été proposées pour faire disparaître ces taches, et blanchir la peau en lui donnant une coloration uniforme. L'utilisation de nombreuses substances d'origine animale, minérale, ainsi que d'une grande variété de plantes, et de substances d'origine végétale a été préconisée de la sorte. La publication de SCARPA et GUERCI [Journal of Ethnopharmacology, 19 17-66 (1987)] met en évidence la diversité des préparations proposées.

Toutefois, un très petit nombre des recettes ainsi proposées ont fait la preuve réelle de leur efficacité. Actuellement, on ne connaît que très peu de substances ayant un réel effet dépigmentant, et parmi ces substances, la plupart présentent des effets secondaires gênants, voire néfastes.

Par exemple, certaines préparations à base de sels de mercure, bien que dotées d'un réel pouvoir éclaircissant sur la peau, présentent des dangers réels, car le mercure absorbé par voie transcutanée, peut causer de graves intoxications.

La plupart des préparations dépigmentantes commercialisées actuellement contiennent comme principe actif de l'hydroquinone. Ces préparations ne sont toutefois pas dépourvues d'inconvénients. Elles sont en effet d'un maniement très délicat : leur application doit en effet être strictement limitée aux zones à dépigmenter, et la quantité utilisée doit être soigneusement dosée. Ces inconvénients sont particulièrement importants chez des sujets à peau foncée, qui risquent l'apparition de nouvelles taches, résultant d'une dépigmentation non uniforme. En outre l'hydroquinone est mal supportée par de nombreux sujets, chez qui elle provoque des irritations de la peau.

Parmi les innombrables principes actifs d'origine végétale, ou extraits de plantes, auxquels une action dépigmentante a été attribuée, un seul est effectivement utilisé en cosmétologie : il s'agit d'un extrait d'achillée millefeuille, dont l'activité proviendrait de la présence de lutéoline et de ses hétérosides.

L'Inventeur a entrepris des recherches dans le but de découvrir d'autres substances régulant la pigmentation sans toutefois présenter les inconvénients des substances actuellement utilisées.

Il est en effet souhaitable de disposer de substances faisant disparaître les taches pigmentaires, sans pour autant provoquer un blanchissement général de la peau ou bien une dépigmentation excessive des zones traitées. Il faut en outre que ces principes actifs soient bien tolérés, et en particulier, ne provoquent d'irritation.

Or, l'Inventeur a constaté que des extraits obtenus à partir de la plante *Eclipta alba* possédaient des propriétés dépigmentantes, sans provoquer les effets secondaires mentionnés ci-dessus. En fractionnant les extraits obtenus, il a en outre constaté que ces propriétés étaient associées à la fraction riche en flavonoïdes de la plante, et que des préparations comprenant de la wedelolactone ou de la desméthylwedelolactone qui sont les principaux flavonoïdes d'*Eclipta alba*, possédaient également des propriétés dépigmentantes.

*Eclipta alba* est une plante qui appartient à la famille des, composées, et qui est fréquemment rencontrée dans les régions tropicales (Inde, Afrique, Antilles, Guyane). Cette plante a fait l'objet de diverses études qui ont abouti à la mise en évidence de différents principes actifs. C'est ainsi que BHARGAVA et al. [Indian Journal of Chemistry, 10 810-811 (1972)] ont extrait des feuilles 3 isoflavonoïdes : la wedelolactone, la desméthylwedelolactone et son glucoside. QUISUMBING [Medicinal Plants of the Philippines : Technical Bulletin - Departement of Agriculture and Natural Resources - Republic of the Philippines, Manila ; 16, 1234 (1951)] a mis en évidence des résines, ainsi qu'un alcaloïde : l'écliptine. KARRER [Konstitution und Vorkommen der Organischen Pflanzenstoffe, Birkhäuser Verlag - Bascl et Stuttgart, 1er Vol. p. 1207, (1958); supplément I, p. 1038, (1977) ; Supplément II p. 939 (1981)] a décrit la présence d'un stérol : le stigmastérol. HEYWOOD et al. [The Biology and Chemistry of the Compositae - Academic Press, London (1977)] ont indiqué que les parties aériennes renfermaient de la nicotine.

La médecine traditionnelle a proposé différentes utilisations d'*Eclipta alba* ; en particulier, aux Antilles, les racines pilées ont été utilisées comme anti-hémorragique, ainsi que dans le traitement de la lèpre. Certains travaux ont conduit à attribuer à cette plante une activité anti-virale [DHAR et al., Indian J. Exp. Biology, . 232-247, (1968)], et plus récemment,, une certaine activité anti-bactérienne *in vitro* [PHADKE S.A., Indian J. Med. Sci. 43:(5), 113-117, (1989)]. Une revue des différentes utilisations connues a été publiée par H.M.BURKILL ["The Useful Plants of West Tropical Africa", vol 1, 466-468, Royal Botanic Garden Kews, (1985)].

Toutefois, jusqu'à présent, aucune activité d'*Eclipta alba* sur les défauts de pigmentation de la peau n'avait été montrée.

La présente Invention a pour objet des compositions cosmétiques, régulatrices de la pigmentation cutanée, caractérisées en ce qu'elles comprennent en tant que principe actif, de la wedelolactone et/ou de la desméthylwedelolactone .

Selon un mode de réalisation préféré des compositions cosmétiques conformes à l'Invention, elles comprennent entre 0,1 et 15% en poids de wedelolactone et/ou entre 0,1 et 15% en poids de desméthylwedelolactone.

Des compositions faiblement dosées (moins de 0,5% de chacun des principes actifs) seront de préférence utilisées à titre préventif, ou en traitement d'entretien. Les compositions plus fortement dosées (entre 0,5 et 2%) seront utilisées de préférence pour le traitement des défauts de la pigmentation déjà installés. Les compositions les plus fortement dosées (plus de 2%) seront préférées en traitement d'attaque, en particulier dans le cas des irrégularités de la pigmentation, d'installation ancienne. Dans certains cas particuliers, des compositions encore plus fortement dosées peuvent être utilisées sans inconvénient, grâce à l'excellente tolérance de ces principes actifs. L'Inventeur a ainsi testé avec succès un extrait hydroalcoolique d'*Eclipta alba* concentré, comprenant 10% de desméthylwedelolactone et 7% de wedelolactone, sans observer d'effets secondaires autres qu'un léger dessèchement de la peau, imputable à la teneur en alcool de l'extrait.

Selon un mode de réalisation préféré des compositions cosmétiques conformes à l'Invention, ledit principe actif est présent sous forme d'un extrait de plantes riches en wedelolactone et/ou en desméthylwedelolactone .

La présence de wedelolactone et de desméthylwedelolactone en quantité importante a été décrite dans différentes plantes, appartenant en particulier à la famille des Composées, par exemple les *Wedelia* ; des extraits de ces plantes peuvent en conséquence être employés pour l'obtention des compositions conformes à l'invention ; toutefois, au cours des expérimentations effectuées par l'inventeur, les meilleurs résultats ont été obtenus à partir d'un extrait d'*Eclipta alba*.

Selon une disposition préférée de ce mode de réalisation, ledit extrait végétal est un extrait d'*Eclipta alba* ; avantageusement, il s'agit d'un extrait enrichi en flavonoïdes.

Un extrait enrichi en flavonoïdes est, par exemple un extrait hydroalcoolique obtenu à partir de feuilles d'*Eclipta alba*, éventuellement concentré, de façon à comprendre de préférence entre 5 et 25% en poids de desméthylwedelolactone et entre 2 et 15% en poids de wedelolactone.

Avantageusement, les compositions cosmétiques conformes à l'Invention comprennent entre 1 et 25 % en poids d'extrait hydroalcoolique de feuilles d'*Eclipta alba*.

L'extrait d'*Eclipta alba* peut être associé à différents excipients, ainsi qu'à d'autres principes actifs, connus en eux-mêmes, pour obtenir des préparations adaptées aux différents types de peaux, et pouvant associer éventuellement, à l'action dépigmentante, une autre action traitante (hydratants, antirides, adoucissants, anti-séborrhéiques, etc...) .

Les préparations cosmétiques conformes à l'Invention, sont particulièrement efficaces sur les hyperpigmentations mélaniques brunes ou noires cicatricielles, ou provoquées par un agent photosensibilisant, ainsi que sur les hyperpigmentations du lentigo sénile.

Avantageusement, pour obtenir des préparations cosmétiques plus particulièrement destinées au traitement des taches de rousseur ou des hyperpigmentations hormonales (chloasma) on associe aux compositions conformes à l'invention un extrait de *Betula alba*.

Le bouleau (*Betula alba*) fait partie des plantes qui sont traditionnellement préconisées pour éclaircir la peau, sans qu'aucune étude réelle de son efficacité n'ait toutefois été été effectuée. Or, l'Inventeur a constaté que l'activité des compositions conformes à l'Invention sur les hyperpigmentations du type taches de rousseur ou chloasma était considérablement renforcée lorsqu'une préparation à base de sève de bouleau leur était associée.

Les excipients utilisés dans l'obtention des préparations conformes à l'Invention peuvent être très variés, et seront choisis en fonction du type de préparation qu'on souhaite obtenir : lait, lotion, crème, gel, pommade, crème solaire, fond de teint, lait corporel, etc...

L'utilisation régulière des préparations régulatrices de la pigmentation cutanée conformes à l'Invention permet la disparition progressive des défauts de pigmentation et l'obtention d'une teinte uniforme de la peau, et ne présente aucun des inconvénients des préparations de l'art antérieur, en particulier celles à base d'hydroquinone. Au contraire les préparations conformes à l'Invention sont parfaitement tolérées et peuvent être utilisées pendant de longues périodes, voire à titre préventif, sans qu'aucune précaution particulière d'emploi soit nécessaire.

La présente Invention sera mieux comprise à l'aide du complément de description qui va suivre qui se réfère à des exemples de préparation et d'utilisation des compositions dépigmentantes conformes à l'Invention.

Il va de soi toutefois que ces exemples sont donnés uniquement à titre d'illustration de l'objet de l'Invention, dont ils ne constituent en aucune manière une limitation.

### EXEMPLE 1 - OBTENTION D'UN EXTRAIT HYDROALCOOLIQUE A PARTIR DE FEUILLES D'ECLIPTA ALBA

250 g de feuilles d'*Eclipta alba* sont extraits, par macération pendant 48 heures, dans 1000 g d'éthanol 70 °C, en présence de 2% d'acide acétique. Après filtration, la solution est concentrée par évaporation jusqu'à 1/3 de son volume de départ.

Cette solution concentrée contient, pour 100g, 9 g de desméthylwedelolactone, et 6 g de wedelolactone.

### EXEMPLE 2 - OBTENTION D'UN EXTRAIT DE BETULA ALBA

20 g de sève de bouleau sont mis en solution dans 100 g d'alcool à 30 °C.

### EXEMPLE 3 - PREPARATION DE COMPOSITIONS COSMETIQUES CONFORMES A L'INVENTION

### A) CREME FLUIDE

| | |
|---|---|
| . Vaseline fluide | 8 g |
| . Perhydrosqualène | 5 g |
| . Téfose 2561 (GATTEFOSSE) | 10 g |
| . Arlamol HD (ICI) | 6 g |
| . Arlamol D4 (ICI) | 2 g |
| . Carbopol 940 (POLYPLASTIC) | 0,3 g |
| . *Eclipta alba* (extrait concentré) | 5 g |
| . Parahydroxybenzoate de méthyle | 0,02 g |
| . Parahydroxybenzoate de propyle | O,01 g |
| . Triethanolamine | 0,3 g |
| . Filtre Ensolex 8021 (MERCK) | 2 g |
| . Eau | QSP 100 g |

### B) CREME EPAISSE

| | |
|---|---|
| . Vaseline fluide | 8 g |
| . Perhydrosqualène | 5 g |
| . Téfose 2561 (GATTEFOSSE) | 10 g |
| . Arlamol HD (ICI) | 6 g |
| . Arlamol D4 (ICI) | 2 g |
| . Carbopol 940 (POLYPLASTIC) | 0,3 g |
| . *Eclipta alba* (extrait concentré) | 15 g |
| . *Betula alba* (extrait) | 5 g |
| . Triethanolamine | 0,3 g |
| . Parahydroxybenzoate de méthyle | 0,02 g |
| . Parahydroxybenzoate de propyle | O,01 g |
| . Eau | QSP 100 g |

### C) PROCEDE DE PREPARATION

I) Disperser le carbopol dans 2/3 de la quantité totale d'eau. Laisser gonfler ;
II) Peser et mettre à dissoudre au bain marie la vaseline fluide, le perhydrosqualène, le Téfose, l'arlamol HD ;
III) Peser les principes actifs (extrait d'*Eclipta alba* et/ou extrait de *Betula alba*), y dissoudre les parahydroxybenzoate de méthyle et propyle ;
IV) Peser la triéthanolamine et la dissoudre dans les 1/3 d'eau restante ;
V) Peser l'Arlamol D4 ;

Après avoir porté les mélanges I) et II) à 90°C, mélanger à la turbine.
A 40°C ajouter le mélange III) puis l'arlamol D4 et ensuite neutraliser en ajoutant le mélange IV).

### D) GEL

| | |
|---|---|
| . Carbopol 940 | 0,5% |
| . Triethanolamine | 0,5% |
| . *Eclipta alba* (extrait concentré) | 10% |
| . Eau | QSP 100 |

### EXEMPLE 4 - UTILISATION DES COMPOSITIONS COSMETIQUES CONFORMES A L'INVENTION

Une expérimentation a été réalisée sur 3 sujets présentant un chloasma, 2 sujets présentant des taches de rousseur, 4 sujets présentant une hyperpigmentation cicatricielle consécutive à une acné, 3 sujets présentant un lentigo sénile. La formulation décrite à l'exemple 3-B, à l'exception de l'extrait de *Betula alba*, a été appliquée biquotidiennement sur les zones à traiter, pendant 2 mois.

Aucune protection solaire particulière n'est utilisée pendant le traitement.
Les résultats sont les suivants :
Chez les sujets présentant une hyperpigmentation cicatricielle, ainsi que chez ceux présentant un lentigo sénile, on observe dès la troisième semaine de traitement, une atténuation des taches et une unification de la couleur de la peau. Au bout de deux mois de traitement, l'hyperpigmentation cicatricielle a quasiment disparu chez 3 sujets sur 4, et est très fortement atténuée chez le quatrième. Les taches de lentigo sont très atténuées et la coloration de la peau unifiée, chez les 3 sujets traités.
Chez les sujets présentant des taches de rousseur et ceux présentant un chloasma, on observe une légère atténuation à la fin des deux mois de traitement. Le traitement est poursuivi pendant 2 mois chez ces sujets, en utilisant la formulation de l'exemple 3-B complète (c'est-a-dire, comprenant l'extrait de *Betula alba*).
A l'issu de ces 2 mois supplémentaires de traitement, on observe une très nette atténuation du chloasma chez les 3 sujets traités et même une disparition presque totale chez l'un d'entre eux, et une atténuation significative des taches de rousseur (uniformisation visible de l'aspect du teint) chez les 2 sujets concernés.

## Revendications (Revendications pour l'(les) Etat(s) contractant(s) suivant(s): AT, BE, CH, DE, FR, GB, IT, LI, LU, NL)

1. Composition cosmétique régulatrice de la pigmentation, adaptée à l'application cutanée, caractérisée en ce qu'elle comprend en tant que principe actif, de la wedelolactone et/ou de la desméthylwedelolactone.

2. Composition cosmétique selon la Revendication 1, caractérisée en ce qu'elle comprend entre 0,1 et 15% en poids de wedelolactone et/ou entre 0,1 et 15% en poids de desméthylwedelolactone.

3. Composition cosmétique selon l'une quelconque des Revendications 1 ou 2, caractérisée en ce que ledit principe actif est présent sous forme d'un extrait de plantes riche en wedelolactone et/ou en desméthylwedelolactone.

4. Composition cosmétique selon la Revendication 3, caractérisée en ce que ledit principe actif est présent sous forme d'un extrait hydroalcoolique obtenu à partir de feuilles d'*Eclipta alba*.

5. Composition cosmétique selon la Revendication 4, caractérisée en ce qu'elle comprend entre 1 et 25% d'extrait hydroalcoolique de feuilles d'*Eclipta alba*.

6. Composition cosmétique selon l'une quelconque des revendications 1 à 5, caractérisée en ce qu'elle comprend en outre un extrait de *Betula alba*.

7. Utilisation de la wedelolactone et/ou de la desméthylwedelolactone, et/ou d'un extrait végétal contenant au moins l'un desdits principes actifs, pour l'obtention d'une composition cosmétique régulatrice de la pigmentation cutanée.

8. Utilisation de la wedelolactone et/ou de la desméthylwedelolactone, et/ou d'un extrait végétal contenant au moins l'un desdits principes actifs, à titre de produit cosmétique régulateur de la pigmentation cutanée.

9. Utilisation selon la revendication 8 caractérisée en ce que ledit extrait végétal est un extrait d'*Eclipta alba*.

10. Utilisation selon la revendication 8 caractérisée en ce que wedelolactone et/ou la desméthylwedelolactone, sont utilisées sous forme d'une composition selon une quelconque des Revendications 1 à 6.

## Revendications (Revendications pour l'(les) Etat(s) contractant(s) suivant(s): ES, GR)

1. Procédé d'obtention d'une composition cosmétique régulatrice de la pigmentation, et adaptée à l'application cutanée, caractérisé en ce que l'on incorpore de la wedelolactone et/ou de la desméthylwedelolactone, en tant que principe actif, à ladite composition cosmétique.

2. Procédé selon la Revendication 1, caractérisé en ce que l'on incorpore entre 0,1 et 15% en poids de wedelolactone et/ou entre 0,1 et 15% en poids de desméthylwedelolactone.

3. Procédé selon l'une quelconque des Revendications 1 ou 2, caractérisé en ce que ledit principe actif est incorporé sous forme d'un extrait de plantes riche en wedelolactone et/ou en desméthylwedelolactone.

4. Procédé selon la Revendication 3, caractérisé en ce que ledit principe actif est incorporé sous forme d'un extrait hydroalcoolique obtenu à partir de feuilles d'*Eclipta alba*.

5. Procédé selon la Revendication 4, caractérisé en ce que l'on incorpore entre 1 et 25% d'extrait hydroalcoolique de feuilles d'*Eclipta alba*.

6. Procédé selon l'une quelconque des revendications 1 à 5, caractérisé en ce que l'on incorpore en outre un extrait de *Betula alba*.

7. Procédé cosmétique de régulation de la pigmentation cutanée, caractérisé en ce qu'il met en oeuvre de la wedelolactone et/ou de la desméthylwedelolactone, et/ou un extrait végétal contenant au moins l'un desdits principes actifs.

8. Procédé selon la revendication 7 caractérisé en ce que ledit extrait végétal est un extrait d'*Eclipta alba*.

9. Procédé selon la revendication 7 caractérisé en ce que la wedelolactone et/ou la desméthylwedelolactone sont utilisées sous forme d'une composition obtenue par un procédé selon une quelconque des Revendications 1 à 6.

## Claims (Claims for the following Contracting State(s): AT, BE, CH, DE, FR, GB, IT, LI, LU, NL)

1. Cosmetic composition for the regulation of pigmentation, suitable for cutaneous application, characterized in that it comprises, as active principle, wedelolactone and/or demethylwedelolactone.

2. Cosmetic composition according to Claim 1, characterized in that it comprises between 0.1 and 15% by weight of wedelolactone and/or between 0.1 and 15% by weight of demethylwedelolactone.

3. Cosmetic composition according to either of Claims 1 and 2, characterized in that the said active principle is present in the form of a plant extract which is rich in wedelolactone and/or in demethylwedelolactone.

4. Cosmetic composition according to Claim 3, characterized in that the said active principle is present in the form of an aqueous-alcoholic extract obtained from leaves of *Eclipta alba*.

5. Cosmetic composition according to Claim 4, characterized in that it comprises between 1 and 25% of an aqueous-alcoholic extract of leaves of *Eclipta alba*.

6. Cosmetic composition according to any one of Claims 1 to 5, characterized in that it comprises in addition an extract of *Betula alba*.

7. Use of wedelolactone and/or of demethylwedelolactone, and/or of a plant extract containing at least one of the said active principles, to obtain a cosmetic composition for the regulation of cutaneous pigmentation.

8. Use of wedelolactone and/or of demethylwedelolactone and/or of a plant extract containing at least one of the said active principles, by way of a cosmetic product for the regulation of cutaneous pigmentation.

9. Use according to Claim 8, characterized in that the said plant extract is an extract of *Eclipta alba*.

10. Use according to Claim 8, characterized in that wedelolactone and/or demethylwedelolactone are used in the form of a composition according to any one of Claims 1 to 6.

## Claims (Claims for the following Contracting State(s): ES, GR)

1. Process for obtaining a cosmetic composition for the regulation of pigmentation and suitable for cutaneous application, characterized in that wedelolactone and/or demethylwedelolactone are incorporated into the said cosmetic composition by way of active principle.

2. Process according to Claim 1, characterized in that between 0.1 and 15% by weight of wedelolactone and/or between 0.1 and 15% by weight of demethylwedelolactone is incorporated.

3. Process according to either of Claims 1 and 2, characterized in that the said active principle is incorporated in the form of a plant extract which is rich in wedelolactone and/or in demethylwedelolactone.

4. Process according to Claim 3, characterized in that the said active principle is incorporated in the form of an aqueous-alcoholic extract obtained from leaves of *Eclipta alba*.

5. Process according to Claim 4, characterized in that between 1 and 25% of an aqueous-alcoholic extract of leaves of *Eclipta alba* is incorporated.

6. Process according to any one of Claims 1 to 5, characterized in that an extract of Betula alba is also incorporated.

7. Cosmetic process for the regulation of cutaneous pigmentation, characterized in that it uses wedelolactone and/or demethylwedelolactone, and/or a plant extract containing at least one of the said active principles.

8. Process according to Claim 7, characterized in that the said plant extract is an extract of *Eclipta alba*.

9. Process according to Claim 7, characterized in that wedelolactone and/or demethylwedelolactone are used in the form of a composition obtained by the process according to any one of Claims 1 to 6.

## Patentansprüche (Patentansprüche für folgende(n) Vertragsstaat(en): AT, BE, CH, DE, FR, GB, IT, LI, LU, NL)

1. Der Anwendung auf die Haut angepaßte kosmetische Zusammensetzung zur Regulierung der Pigmentierung, dadurch **gekennzeichnet,** daß sie als Wirkstoff Wedelolacton und/oder Desmethylwedelolacton umfaßt.

2. Kosmetische Zusammensetzung nach Anspruch 1, dadurch **gekennzeichnet,** daß sie zwischen 0,1 und 15 Gew.-% Wedelolacton und/oder zwischen 0,1 und 15 Gew.-% Desmethylwedelolacton umfaßt.

3. Kosmetische Zusammensetzung nach einem der Ansprüche 1 oder 2, dadurch **gekennzeichnet,** daß der Wirkstoff in Form eines Extrakts von an Wedelolacton und/oder an Desmethylwedelolacton reichen Pflanzen vorliegt.

4. Kosmetische Zusammensetzung nach Anspruch 3, dadurch **gekennzeichnet,** daß der Wirkstoff in Form eines wäßrig-alkoholischen Extrakts, erhalten aus Eclipta alba-Blättern, vorliegt.

5. Kosmetische Zusammensetzung nach Anspruch 4, dadurch **gekennzeichnet,** daß sie zwischen 1 und 25% des wäßrig-alkoholischen Extrakts aus Eclipta alba-Blättern umfaßt.

6. Kosmetische Zusammensetzung nach einem der Ansprüche 1 bis 5, dadurch **gekennzeichnet,** daß sie weiterhin einen Betula alba-Extrakt umfaßt.

7. Verwendung von Wedelolacton und/oder Desmethylwedelolacton und/oder eines Pflanzenextrakts, der mindestens einen der Wirkstoffe enthält, zur Herstellung einer kosmetischen Zusammensetzung zur Regulation der Hautpigmentierung.

8. Verwendung von Wedelolacton und/oder Desmethylwedelolacton und/oder eines pflanzlichen Extrakts, der mindestens einen der Wirkstoffe enthält, als kosmetisches Produkt zur Regulation der Hautpigmentierung.

9. Verwendung nach Anspruch 8, dadurch **gekennzeichnet,** daß der Pflanzenextrakt ein Extrakt von Eclipta alba ist.

10. Verwendung nach Anspruch 8, dadurch **gekennzeichnet,** daß Wedelolacton und/oder Desmethylwedelolacton in Form einer Zusammensetzung nach einem der Ansprüche 1 bis 6 verwendet werden.

## Patentansprüche (Patentansprüche für folgende(n) Vertragsstaat(en): ES, GR)

1. Verfahren zur Herstellung einer der Anwendung auf die Haut angepaßten kosmetischen Zusammensetzung zur Regulation der Pigmentierung, dadurch **gekennzeichnet,** daß man Wedelolacton und/oder Desmethylwedelolacton als Wirkstoff in die kosmetische Zusammensetzung einarbeitet.

2. Verfahren nach Anspruch 1, dadurch **gekennzeichnet,** daß man zwischen 0,1 und 15% Gew.-% Wedelolacton und/oder zwischen 0,1 und 15 Gew.-% Desmethylwedelolacton einarbeitet.

3. Verfahren nach einem der Ansprüche 1 oder 2, dadurch **gekennzeichnet,** daß der Wirkstoff in Form eines Extrakts von an Wedelolacton und/oder an Desmethylwedelolacton reichen Pflanzen eingearbeitet wird.

4. Verfahren nach Anspruch 3, dadurch **gekennzeichnet,** daß der Wirkstoff in Form eines wäßrigalkoholischen Extrakts, erhalten aus Eclipta alba-Blättern, eingearbeitet wird.

5. Verfahren nach Anspruch 4, dadurch **gekennzeichnet,** daß zwischen 1 und 25% des wäßrigalkoholischen Extrakts von Eclipta alba-Blättern eingearbeitet werden.

6. Verfahren nach einem der Ansprüche 1 bis 5, dadurch **gekennzeichnet,** daß man weiterhin einen Extrakt von Betula alba einarbeitet.

7. Kosmetisches Verfahren zur Regulation der Hautpigmentierung, dadurch **gekennzeichnet,** daß man Wedelolacton und/oder Desmethylwedelolacton und/oder einen Pflanzenextrakt, der mindestens einen der Wirkstoffe enthält, einsetzt.

8. Verfahren nach Anspruch 7, dadurch **gekennzeichnet,** daß der Pflanzenextrakt ein Extrakt von Eclipta alba ist.

9. Verfahren nach Anspruch 7, dadurch **gekennzeichnet,** daß Wedelolacton und/oder Desmethylwedelolacton in Form einer Zusammensetzung, erhalten nach einem Verfahren nach einem der Ansprüche 1 bis 6, verwendet werden.
